# EUROPÄISCHE PATENTSCHRIFT

(11) **EP 3 354 636 B1**
(45) Veröffentlichungstag und Bekanntmachung des Hinweises auf die Patenterteilung: **10.03.2021**
(21) Anmeldenummer: 17153047.0
(22) Anmeldetag: 25.01.2017
(51) Int. Cl.: C07C 209/60

(54) **VERFAHREN ZUR HYDROAMINOMETHYLIERUNG ZYKLISCHER DIENE ZU DI-SEKUNDÄREN AMINEN**
METHOD FOR THE HYDROAMINOMETHYLATION OF CYCLIC DIENES TO DI-SECONDARY AMINES
PROCÉDÉ D'HYDRO-AMINOMÉTHYLATION DE DIÈNES CYCLIQUES EN AMINES SECONDAIRES DI

(43) Veröffentlichungstag der Anmeldung: 01.08.2018
(73) Patentinhaber: OQ Chemicals GmbH, 40789 Monheim am Rhein (DE)
(72) Erfinder: Fuchs, Sarah, 44137 Dortmund (DE); Vorholt, Andreas Johannes, 44137 Dortmund (DE); Behr, Arno, 44137 Dortmund (DE); Meier, Gregor, 47057 Duisburg (DE); Strutz, Heinz, 47445 Moers (DE)
(74) Vertreter: Michalski Hüttermann & Partner Patentanwälte mbB

(56) Entgegenhaltungen:
- DE-A1- 2 363 324
- DE-A1- 19 608 559
- DE-T2- 69 317 334
- US-A- 4 250 115
- US-A- 4 448 996

## Beschreibung

Die Erfindung betrifft ein Verfahren zur Umsetzung zyklischer Diene zu Di-sekundären Aminen, wobei zyklische Diene zusammen mit primären Aminen und Synthesegas an einem organophosphor-freien Metallkatalysator, der ausschließlich Rhodium als Katalysatormetall enthält, zur Reaktion gebracht werden.

Amine gehören zu den wichtigen Zwischenprodukten im Bereich der Groß- und Feinchemikalien mit einer weltweiten Syntheseleistung von mehreren Millionen Jahrestonnen. Anwendungstechnischen Einsatz finden die Amine zumeist in der Farbstoffindustrie, zur Herstellung von Pharmazeutika, als Additive für Schmieröle und Dieselkraftstoffe sowie im Agrobereich.

In der Literatur findet sich eine Vielzahl unterschiedlicher Synthesewege zur Herstellung dieser Substanzklasse, welches einerseits die wirtschaftliche Bedeutung und andererseits die Komplexität der Umsetzung widerspiegelt.

Zur Herstellung aliphatischer Amine werden in der Literatur beispielsweise die nukleophile Substitution von Alkylhaliden, Umsetzungen von Alkenen mit Blausäure inklusiver anschließender Reduktion sowie reduktive Aminierungen von Carbonylverbindungen beschrieben.

Ein weiterer gangbarer Weg ist die Darstellung sekundärer Amine über eine Hydroaminomethylierung ungesättigter Verbindungen. In dieser mehrstufigen Umsetzung reagieren Olefine unter Vergrößerung des C-Gerüstes mit Synthesegas und Aminen metallkatalysiert zuerst zu Aldehyden und anschließend, über eine Imin-Zwischenstufe, zu den entsprechenden Aminen. Das Prinzip der Umsetzung ist in folgendem Reaktionsschema 1 widergegeben:

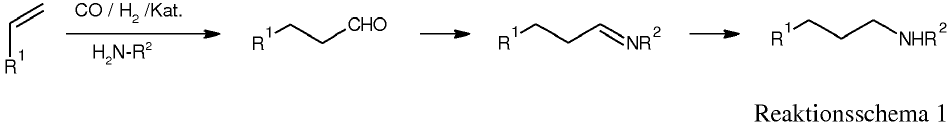

Die Reaktionsführung ist komplex, da bedingt durch die Mehrstufigkeit verschieden funktionale Intermediate mit unterschiedlichen Reaktivitäten auftreten, welche neben dem gewünschten Produkt auch eine Vielzahl unerwünschter Nebenprodukte liefern können. Beispielhaft genannt seien das Auftreten unerwünschter Polymerisations- und Aldolreaktionen der End- sowie Zwischenprodukte.

Insbesondere für "Eintopfumsetzungen", in welchem die oben dargestellte Reaktionskette in nur einer Reaktionslösung und überwiegend zur selben Zeit abläuft, sind hohe Umsetzungsgrade und hohe Selektivitäten schwer zu erreichen, da die Effizienz der Einzelreaktionen nicht unabhängig voneinander optimiert werden kann.

Ein mögliches einstufiges Verfahren zur Hydroaminomethylierung beschreibt beispielsweise DE 19737053 A1. Dieses Dokument offenbart eine einstufige Umsetzung eines C2-C20-Olefins mit einer eine NH₃-Gruppe aufweisenden Verbindung, Wasserstoff und Kohlenmonoxid, dadurch gekennzeichnet, dass (a) die Umsetzung in Gegenwart eines Katalysators stattfindet, der in Wasser in gelöster oder suspendierter Form vorliegt; und (b) der Katalysator mindestens zwei Metalle aus der Gruppe VIII des Periodensystems der Elemente in elementarer oder gebundener Form enthält.

Einen anderen Syntheseweg gibt die DE 10 2013 201668 A1 an. Diese Anmeldung lehrt ein Verfahren zur Hydroaminomethylierung von Olefinen, wobei wenigstens ein Olefin mit mindestens einem primären oder sekundären Amin unter Zuführung von H₂ und CO in Gegenwart eines katalytischen Systems aus einem Ruthenium-Komplex und mindestens einem Liganden umgesetzt wird, wobei der Ligand einen organischen Phosphit-Liganden darstellt.

Das US-Patent 4,250,115 offenbart die Herstellung tertiärer Amine durch Umsetzung eines Olefins, Kohlenmonoxid, Wasserstoff und eine ein Stickstoffatom enthaltende Verbindung wie beispielsweise Ammoniak, einem primären oder einem sekundären Amin in Gegenwart eines rhodium- oder rutheniumhaltigen Katalysators bei Temperaturen im Bereich von etwa 50° bis etwa 350° C und einem Druck im Bereich von ca. 10 bis ca. 300 Atmosphären.

Die DE 693 17 334 T2 offenbart ein Verfahren zur Herstellung eines sekundären Amins, welches ein Umsetzen eines primären Amins mit Kohlenmonoxid, Wasserstoff und einer olefinischen Verbindung in Anwesenheit einer Katalysatorzusammensetzung umfasst, die eine Quelle für kationisches Rhodium, eine Quelle für ein von einem Halogenid unterschiedliches Anion und, als Träger, ein Polymer einer N-heterocyclischen Verbindung umfasst.

Weitere Verfahren zur Darstellung diverser Amine sind in DE 19 608 559 A1, der DE 2 363 324 und der US 4,448,996 angegeben.

In Anbetracht der Vielzahl an unterschiedlichen Olefinklassen und der wirtschaftlichen Bedeutung der daraus erhältlichen Produkte, besteht weiterhin ein großes Interesse an einfachen, kostengünstigen und effizienten Synthesewegen. Es ist daher die Aufgabe der vorliegenden Erfindung einen solchen Syntheseweg bereitzustellen, welcher in einer Eintopfreaktion, unter möglichst umweltschonenden Reaktionsbedingungen sehr hohe Ausbeuten gewährleistet.

Erfindungsgemäß ist das Verfahren zur Synthese di-sekundärer Amine aus zyklischen Dienen und primären Aminen dadurch gekennzeichnet, dass in nur einer Reaktionslösung zyklische Austauschseite

Diene mit Synthesegas und primären Aminen an einem organophosphor-freien Metallkatalysator, der ausschließlich Rhodium als Katalysatormetall enthält, zur Reaktion gebracht werden, wobei die Reaktionslösung von weiteren stickstoffhaltigen organischen Verbindungen neben den als Edukt eingesetzten primären Aminen und den daraus entstehenden Produkten frei ist. Überraschenderweise wurde gefunden, dass sich mit vorliegender Reaktionsführung Hydroaminomethylierungen selbst an sterisch ungünstigen, zyklischen Olefinen durchführen lassen, wobei sich unter diesen ungünstigen Hydroaminomethylierungsbedingungen sogar zweifache Umsetzungen unterschiedlich am Ring positionierter Doppelbindungen mit hoher Ausbeute ergeben. Insbesondere ist dies für zyklische Verbindungen nicht naheliegend, da davon auszugehen ist, dass die oben beschriebene dreistufige Umsetzung, welche zumindest unter zwingender Wechselwirkung mit dem eingesetzten Metallkatalysator in einer oder mehreren Stufen stattfindet, durch die sterisch rigiden Randbedingungen des Ringgerüstes deutlich behindert wird. Des Weiteren ist davon auszugehen, dass sich die Doppelbindungen aufgrund ihrer unterschiedlichen Position im Ringgerüst in ihrer Reaktivität unterscheiden und so bevorzugt nur eine der Positionen umgesetzt wird. Diese Situation wird noch dadurch kompliziert, dass mit einiger Wahrscheinlichkeit die Reaktivität am katalytischen Zentrum für die Reaktion an der zweiten Doppelbindung durch die an der ersten Doppelbindung eingeführte Funktionalität beeinträchtigt werden kann. Diese Randbedingungen sind a priori einer erfolgreichen Umsetzung zweier Doppelbindungen an zyklischen Strukturen abträglich.

In Anbetracht der möglichen Nebenreaktionen der einzelnen Zwischen- und Endprodukte ist es weiterhin überraschend, dass diese Reaktionen in nur einer Reaktionslösung ohne intermediäre Aufarbeitung oder Anpassungen dieser durchgeführt werden können. Dies bedeutet, dass die durch das Verfahren bereitgestellte chemische Umgebung zur Durchführung sämtlicher Umsetzungsschritte im hohen Maße geeignet ist.

Ein weiterer Verfahrensvorteil ergibt sich zudem dadurch, dass die Reaktion mit einem "einfachen" Katalysatorsystem durchgeführt wird, welches auf komplexe und teure Zusammensetzungen unterschiedlicher Metalle und potentiell wasserbelastende organische Phosphorliganden gänzlich verzichten kann. Insbesondere wird auch die Gefahr von Verschleppungen dieser organischen Liganden ins Zielprodukt gänzlich vermieden. Insbesondere kann es aber auch sein, dass es durch die sterische Ausgestaltung der üblicherweise voluminösen organophosphor Liganden zu einem gehinderten Zutritt der hier verwendeten Edukte an den Katalysator kommt, welches zu einer reduzierten Effizienz des Katalysatorsystems führt.

Das erfindungsgemäße Verfahren führt zur Synthese zyklischer di-sekundärer Amine, welche sich durch das Vorhandensein zweier, nicht benachbarter sekundärer Amingruppen auszeichnen. Die beiden Kohlenwasserstoffreste der Amingruppe ergeben sich als Folge der gewählten Umsetzung und werden einerseits durch den organischen Rest des Einsatzamins und andererseits durch den Aufbau des zyklischen Diens festgelegt. Die Umsetzung zu linearen Diaminen aus azyklischen Dienen ist ebenso wenig im Sinne der Erfindung wie die Synthese di-primärer Amine.

Unter einem zyklischen Dien im Sinne der Erfindung versteht man eine organische Verbindung, welche zumindest eine geschlossene, ringförmige Kohlenwasserstoffeinheit und zwei nicht in aromatische Strukturen eingebundene Doppelbindungen aufweist. Das zyklische Dien kann prinzipiell mehrere Ringstrukturen, bevorzugt bis zu 3 Ringstrukturen, und auch mehr als zwei Doppelbindungen tragen. Der Zyklus kann dabei sowohl aliphatischer wie auch aromatischer Struktur sein. Es sind auch gemischte Strukturen möglich, beispielsweise bi-Zyklen, welche einen aromatischen und einen aliphatischen Zyklus aufweisen. Die Doppelbindungen können dabei sowohl inner- wie auch außerhalb des Zyklus vorliegen. Doppelbindungen aromatischer Zyklen zählen nicht als reaktive Doppelbindungen im Sinne der hier vorgestellten Umsetzung. Des Weiteren ist das hier vorgestellte Verfahren nicht auf die Umsetzung reiner Kohlenwasserstoffdiene beschränkt, wobei es möglich ist, dass das Dien weitere Heteroatome aufweist. Prinzipiell eignen sich aliphatische oder araliphatische zyklische Diene mit einer Kohlenstoffzahl von C4-C30 für das hier vorgestellte Verfahren.

Zur erfindungsgemäßen Aminierung geeignete primäre Amine sind Amine, welche zumindest eine Amingruppe aufweisen, wobei der Organylrest der Amingruppe einen C1-C18 aliphatischen, aromatischen oder araliphatischen Kohlenwasserstoffrest darstellt. Das primäre Amin kann aber auch noch weitere funktionelle Gruppen wie beispielsweise Halogene, Hydroxygruppen, oder weitere Alkylamingruppen tragen.

Die Umsetzung der zyklischen Diene mit den primären Aminen erfolgt in nur einer Reaktionslösung. Dies bedeutet, dass die Umsetzung in nur einer Reaktionsumgebung (z.B. nur einem Reaktor) durchgeführt wird, wobei der Reaktionslösung während der Synthese aktiv keine Substanzen entnommen werden (zum Beispiel im Rahmen einer Reaktivdestillation). Unbenommen davon können aber eine oder mehrere der Reaktionsteilnehmer erst im Laufe der Reaktion ganz oder teilweise zur Reaktionslösung dosiert werden.

Die Reaktion der Diene zu di-sekundären Aminen erfolgt in Gegenwart von Synthesegas. Darunter werden insbesondere CO/H₂-Gemische verstanden, in denen Kohlenmonoxid und Wasserstoff in einem Molverhältnis von 0,1 bis 10 vorliegen kann.

Die Aminomethylierung der Doppelbindungen erfolgt metallkatalysiert unter Verwendung eines organophosphor-freien Katalysators, der ausschließlich Rhodium als Katalysatormetall enthält. Der Einsatz eines solchen Rhodiumkatalysators schließt insbesondere den Einsatz von Mischungen verschiedener Metallkatalysatoren mit unterschiedlichen Metallen als aktivem Zentrum aus. Technisch nicht zu vermeidende Restmengen/Verunreinigungen anderer Metalle, in der Regel < 1 mol% oder bevorzugt <0,1 mol% bezogen auf das eingesetzte Rhodium, gelten als vernachlässigbar. Des Weiteren wird weder ein Metallkatalysator eingesetzt, noch bildet sich dieser innerhalb der Reaktion durch Zugabe geeigneter organischer Liganden zur Reaktionslösung, welcher in seiner Koordinationssphäre organische Phosphorliganden aufweist. Die Reaktionslösung und der Katalysator sind demzufolge beispielsweise frei von Triphenylphosphinen, bidentaten Phosphor-Liganden, wie beispielsweise Xantphos oder BINAP. Bei den eingesetzten Katalysator-Edukten handelt es sich in der Regel um Katalysator-Vorstufen (Katalysator-Precursor), welche erst in Kontakt mit dem Synthesegas zu den aktiven Katalysatoren umgesetzt werden.

Die Reaktionsteilnehmer in der Reaktionslösung werden erfindungsgemäß "zur Reaktion gebracht", wobei zumindest ein Teil der Edukte nach oben genanntem Reaktionsschema I in disekundäre Amine umgewandelt werden. Bevorzugt kann dabei der Umsatz an Dien größer oder gleich 50 %, weiter bevorzugt größer als 75 % und des Weiteren bevorzugt größer als 90 % betragen. Der Umsatz zum di-sekundären Amin kann dabei bevorzugt größer als 20 %, 40 %, 50 % oder 70 % betragen.

In einer bevorzugten Ausführungsform kann die Reaktionslösung von weiteren stickstoffhaltigen organischen Verbindungen neben den als Edukt eingesetzten primären Aminen und den daraus entstehenden Produkten frei sein, um notwendige Koordinationsstellen am Katalysator nicht unnötig zu blockieren. Diese Ausführungsform kann zu einer höheren Umsetzung der erfindungsgemäß einsetzbaren Edukte beitragen.

In einer bevorzugten Ausführungsform des Verfahrens kann die Temperatur innerhalb der Umsetzung größer oder gleich 80°C und kleiner oder gleich 160°C betragen. Innerhalb dieses Temperaturbereiches lassen sich mit relativ kurzen Prozesszeiten und einer geringen Bildung unerwünschter Nebenprodukte, sehr hohe Umsätze an Dien mit sehr hohen Selektivitäten zur Bildung des di-primären Amins erreichen. Tiefere Temperaturen können nachteilig sein, da die Prozesszeiten sich sehr verlängern, wohingegen höhere Temperaturen zu einer Polymerisierung gewünschter Produkte und zur Zersetzung des Katalysators führen können. Eine besonders effiziente Reaktionsführung ergibt sich zudem in einem Temperaturintervall zwischen 100 und 140°C.

In einer weiteren bevorzugten Ausgestaltung des Verfahrens kann das Molverhältnis von eingesetztem primären Amin zu eingesetztem Dien (Amin:Dien) größer oder gleich 2 und kleiner oder gleich 40 betragen. Der Einsatz über-stöchiometrischer Mengen an primären Aminen kann zu einer vollständigeren Umsetzung zum gewünschten di-sekundären Amin beitragen und derart die Selektivität der Reaktion deutlich erhöhen. Sehr hohe Ausbeuten an gewünschtem Diamin ergeben sich insbesondere bei einem Verhältnis von 6 bis 12, wobei noch höhere Aminmengen sich nicht negativ auf Umsatz und Ausbeute der Reaktion auswirken.

Innerhalb eines bevorzugten Aspektes des Verfahrens kann die Reaktion in Gegenwart eines Lösungsmittels durchgeführt werden. Obwohl die eingesetzten Edukte in der Regel eine ausreichend niedrige Viskosität und somit ausreichend hohe Mobilität zum Erhalt einer gut durchmischten Reaktionslösung bereitstellen, kann es vorteilhaft sein, die Reaktion in Gegenwart eines zusätzlichen Lösungsmittels durchzuführen. Dies kann zu höheren Umsätzen und einem geringeren Anteil an Nebenprodukten beitragen, da eine schnellere und homogenere Temperaturverteilung in der Lösung erhalten wird. Bevorzugt kann das molare Verhältnis von Lösungsmittel zu eingesetztem Dien (Lösungsmittel:Dien) größer oder gleich 25 und kleiner oder gleich 250, des Weiteren bevorzugt von größer oder gleich 25 und kleiner oder gleich 200 betragen. Diese Lösungsmittel:Dien-Verhältnisse reduzieren insbesondere auch den Anteil hochpolymerer Nebenprodukte.

Nach einer weiteren, bevorzugten Ausgestaltung des Verfahrens kann als Lösungsmittel ein aprotisch unpolares Lösungsmittel eingesetzt werden. Prinzipielle Vertreter dieser Substanzklassen können Alkane, Benzol und andere Aromaten mit aliphatischen und aromatischen Substituenten, Carbonsäureester, Ether, z. B. Diethylether sein. Bevorzugt kann das Lösungsmittel aus der Gruppe bestehend aus Toluol, Cyclohexan, Methylcyclohexan ausgewählt werden. Der Einsatz dieser Lösungsmittel kann insbesondere dazu beitragen, dass beide Doppelbindungen des Diens zum gewünschten Diamin abreagieren. Der Einsatz anderer Lösungsmittel, wie beispielsweise polarer Lösungsmittel, kann dazu führen, dass sich die Ausbeute an gewünschtem Diamin in Richtung der Synthese nur des Monoamins verschiebt.

In einer bevorzugten Charakteristik des Verfahrens kann der Druck innerhalb der Umsetzung größer oder gleich 3 MPa und kleiner oder gleich 30 MPa betragen. Dieser Druckbereich kann zu einer schnellen und hochselektiven Umsetzung der eingesetzten Diene zu den gewünschten Diaminen beitragen. Dieser Druckbereich ergibt sich gegebenenfalls nach mehrmaligem Aufpressen des Synthesegases auf das System und ist für den nicht temperierten Reaktor angegeben (T = 20°C). In einer weiter bevorzugten Ausführungsform kann der Druck größer oder gleich 4 MPa und kleiner oder gleich 25 MPa betragen.

In einer weiteren Ausgestaltung können als Diene C5-C20 Diene mit mindestens einer zyklischen Struktur eingesetzt werden. Insbesondere diese niedermolekularen Diene lassen sich mit dem erfindungsgemäßen Verfahren mit hohen Umsätzen und hoher Selektivität zu den gewünschten Diaminen umsetzen. Ohne durch die Theorie gebunden zu sein, scheinen insbesondere die nicht organophosphor-liganden modifizierten Katalysatoren sterisch sehr gut mit dieser Gruppe zyklischer Diene wechselwirken zu können, sodass sich innerhalb kurzer Prozesszeiten mit hoher Selektivität die gewünschten Diamine bilden können.

Innerhalb einer bevorzugten Ausführungsform können die Diene aus der Gruppe bestehend aus 1,4-Cyclohexadien, 4-Vinyl-1-cyclohexen, 5-Vinyl-2-Norbonene, Norbornadien, Divinylbenzol, Dicyclopentadien, Tricyclopentadien oder Mischungen daraus ausgesucht sein. Diese Gruppe an Dienen zeigt, anscheinend aufgrund ihrer Größe und sterischen Ausgestaltung, sehr hohe Umsätze und ausgezeichnete Selektivitäten im Rahmen der erfindungsgemäßen Umsetzung. Des Weiteren zeigt sich insbesondere ein geringer Anteil an hochsiedenden Nebenprodukten, welche sich nur schwerlich für andere Verwendungszwecke eignen oder mittels weiterer Aufbereitungsmethoden umsetzen lassen.

In einer weiteren Ausgestaltung kann das Dien nur einen aliphatischen Zyklus aufweisen, wobei die Doppelbindungen sich entweder nur im Zyklus oder eine Doppelbindung sich innerhalb- und eine Doppelbindung sich außerhalb der zyklischen Struktur befindet.

In einer weiteren Ausgestaltung kann das Dien nur eine aromatische Ringstruktur aufweisen, wobei die beiden Doppelbindungen sich außerhalb des aromatischen Zyklus befinden.

Nach einem bevorzugtem Verfahrensaspekt können als Diene bi-zyklische C7-C20 Diene eingesetzt werden. Überraschenderweise hat sich gezeigt, dass mit der erfindungsgemäßen Umsetzung auch bi-zyklische Strukturen umgesetzt werden, welche zwei Doppelbindungen aufweisen. Dies ist deshalb überraschend, da die sterischen Bedingungen zur Umsetzung bi-zyklischer im Vergleich zu mono-zyklischen und insbesondere nicht-zyklischen Strukturen schlechter sind. Insbesondere scheint aber der hier gewählte organophosphor-freie Katalysator auch zur Umsetzung dieser Substanzklasse geeignet, und es ergeben sich hohe Umsätze und hohe Selektivitäten.

Innerhalb einer weiteren Ausgestaltung des Verfahrens kann als Dien Dicyclopentadien eingesetzt werden. Insbesondere mit Dicyclopentadien ergeben sich hohe Umsätze, hohe Selektivitäten und nebenproduktarme Umsetzungen.

In einer weiteren bevorzugten Ausführungsform des Verfahrens können die primären Amine aus der Gruppe der primären aliphatischen oder araliphatischen C1-C18 Amine ausgesucht sein. Obwohl prinzipiell die erfindungsgemäße Umsetzung mit einer Vielzahl an primären Aminen gefahren werden kann, scheint die Untergruppe an primären C4-C15 Aminen besonders schnell unter den angegebenen Reaktionsbedingungen reagieren zu können. Es lassen sich bei kurzen Prozesszeiten und moderaten Prozesstemperaturen hoch selektiv und mit guten Ausbeuten die gewünschten di-Amine erhalten. Ohne durch die Theorie gebunden zu sein, liegt dies an der Größe der Amine, welche einen guten Zutritt zum reaktiven Zentrum der zyklischen Diene ermöglicht. Bei den aliphatischen oder araliphatischen Aminen kann es sich im aliphatischen Teil um lineare oder verzweigte Strukturen handeln.

In einer weiteren Verfahrensvariante können die primären Amine aus der Gruppe bestehend aus n-Butylamin, Isobutylamin, Cyclohexylamin, Benzylamin, Isopropylamin, Anilin, sowie Mischungen daraus ausgesucht sein. Mit dieser Gruppe an primären Aminen haben sich selbst bei polyzyklischen Dienen hohe Umsätze und hohe Selektivitäten in der erfindungsgemäßen Umsetzung gezeigt.

In einem weiteren Verfahrensaspekt kann das molare Verhältnis CO zu H₂ (CO:H₂) des eingesetzten Synthesegases größer oder gleich 0,25 und kleiner oder gleich 2 betragen. Diese CO:H₂-Verhältnisse führen zu einer schnellen Reaktion im ersten Verfahrensschritt, der Hydroformulierung, und können bezüglich der Gesamtreaktionsrate oder Produktausbeute einen positiven Einfluss haben.

Innerhalb einer weiteren bevorzugten Ausgestaltung des Verfahrens kann die Vorstufe des Rhodiumkatalysators aus der Gruppe bestehend aus [Rh(octanoat)₂]₂, [Rh(acac)(CO)₂], [Rh(2-Ethylhexanoat)₃], [Rh(2-Ethylhexanoat)₂]₂, [Rh(acac)(cod)], [Rh(cod)Cl]₂, [Rh(acac)₃], [Rh(cod)₂]BF₄ oder Mischungen daraus ausgesucht sein. Diese Gruppe an organophosphor- und nicht produktrelevanten organostickstoff-ligandenfreien Rhodiumkatalysatoren, zeigt für die hier beanspruchten Umsetzung zyklischer Diene hohe Umsatzraten bei niedrigen Temperaturen. Zudem werden durch diese Katalysatoren Umsetzungen erzielt, welche nur einen sehr geringen Anteil polymerer Nebenprodukte aufweisen.

In einer weiteren Charakteristik des Verfahrens kann die molare Menge des Rhodiumkatalysators bezogen auf das Dien (Katalysator:Dien) größer oder gleich 0,0001 mol-% und kleiner oder gleich 1 mol-% betragen. Die erfindungsgemäßen Umsetzungen lassen sich innerhalb kurzer Prozesszeiten auch mit relativ geringen Anteilen an "nackten" Metallkatalysator fahren. Dies kann zur Prozessökonomie beitragen. Weiterhin bevorzugt kann das Katalysator:Dien-Verhältnis größer oder gleich 0,001 mol-% und kleiner oder gleich 0,8 mol-%, des Weiteren größer oder gleich 0,01 mol-% und kleiner oder gleich 0,6 mol-% betragen.

### Beispiele

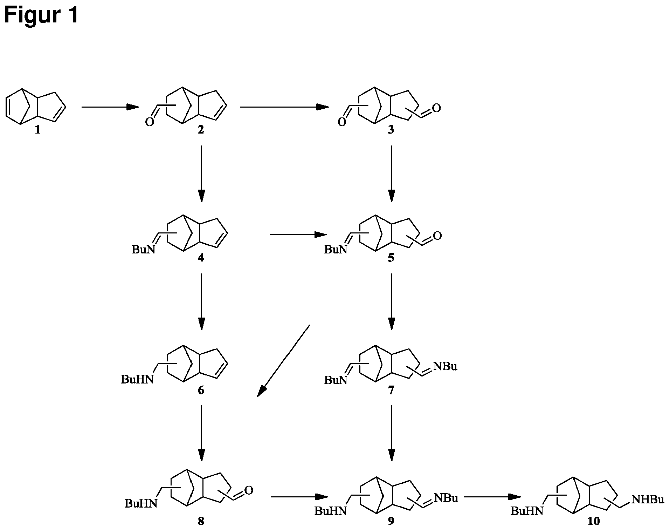

Figur 1 zeigt die Reaktionsstufen einer erfindungsgemäßen Hydroaminomethylierung (HAM) von Dicyclopentadien (DCP, (1)) zu 3(4),8(9)-Bis-(N-(n-butyl)aminomethyl)-tricyclo-[5.2.1.0^{2,6}]decane (TCD-Di(butyl)amin, (10)). Wie aus der Figur ersichtlich, handelt es sich um eine insgesamt 6-stufige Reaktion, wobei jede Doppelbindung des Diens eine 3-stufige Umsetzung durchläuft (Dien zu Aldehyd zu Imin zu Amin).

In der Figur sind mögliche Nebenreaktionen der einzelnen Stufen und Produkte dieser nicht dargestellt. Mögliche, unerwünschte Nebenreaktionen sind beispielsweise die Hydrierung der Doppelbindung und Aldolkondensationen
- zweier Monoaldehyde und
- zweier Dialdehyde.

Weiterhin ist es auch möglich, dass gewünschte Endprodukte mit Zwischenprodukten oder mit sich selbst unter Ausbildung höhermolekularer Oligomere ausreagieren.

Die erste Umsetzung des Diens (1), die Hydroformulierung (HYFO), erfolgt in Gegenwart von Synthesegas und eines nicht organophosphor modifizierten Rhodiumkatalysators vermutlich unter Ausbildung der betreffenden Aldehyde (2, 3). Diese kondensieren dann mit dem primären Amin zu den entsprechenden Iminen (4, 5, 7), welche anschließend zu den Aminen (6, 8, 9, 10) hydriert werden.

### Beispiel 1:

Die Umsetzung erfolgt als "Eintopf-Reaktion" in einem Parr-Reaktor. Es wurden 0,132 g (1mmol) DCP, 0,439 g (6 mmol) n-Butylamin und 0,25 mol% des "nackten" Katalysator-Precursors [Rh(octanoat)₂]₂ bezogen auf das Dien in 5 ml Toluol gelöst und nach Aufpressen des Synthesegases zur Reaktion gebracht. Die Reaktion wurde unter Rühren (500 Umdrehungen pro Minute) für 3 h bei einer Temperatur von 120 °C und einem anfänglichen Synthesegasdruck von 60 bar (Verhältnis CO/H₂ des Synthesegases 1:1) durchgeführt. Man erhält eine vollständige Umsetzung des DCPs mit einer Ausbeute an TCD-di(butyl)amin von 87 % (gemessen mittels GC-FID mit Dibutylether als internen Standard).

Der Einfluss der einzelnen Reaktionsparameter auf die Ausbeute an Diamin wird in den folgenden Punkten dargestellt. Der Einfluss der unterschiedlichen Reaktionsparameter wurde dabei durch Versuche bestimmt, deren Parameterkombinationen mittels statistischer Versuchsplanung festgelegt wurden. Die hier getroffenen qualitativen Aussagen zum Einfluss der einzelnen Reaktionsparameter basieren aber auf statistisch signifikanten Zuordnungen, selbst wenn die zum Vergleich herangezogenen Versuche sich in mehr als einem Reaktionsparameter unterscheiden.

### I. Einsatz unterschiedlicher Dien-Edukte

Das oben angegebene Beispiel wurde mit unterschiedlichen, zyklischen Dienen und n-Butylamin als primäres Amin wiederholt. Der Umsatz der Diene betrug in sämtlichen Fällen 100%. Es ergaben sich folgende Ausbeuten an di-sekundären Aminen:

| Edukt | Ausbeute Diamin in % |
|---|---|
| 5-Vinyl-2-Norbonen | 72 |
| 4-Vinyl-1 -cyclohexen | 62 |
| Norbornadien | 15 |
| 1,4-Cyclohexadien | 12 |
| Divinylbenzol | 62 |

Durch die Umsetzung von Dien-Edukten mit unterschiedlicher Struktur und Lage der Doppelbindungen in der Ringstruktur wurde gezeigt, dass sich unabhängig von der Lage der Doppelbindung, d.h. rein innerhalb, rein außerhalb oder gemischt, mit der erfindungsgemäßen Reaktionsführung doppelte Umsetzungen der Diene erreichen lassen.

### II. Einsatz unterschiedlicher primärer Amine

Das oben angegebene Beispiel wurde mit unterschiedlichen primären Aminen als Edukt wiederholt. Der Umsatz der Diene lag sämtlich bei 100%. Es ergaben sich folgende Ausbeuten an di-sekundären Aminen nach jeweils 1 h Reaktionszeit:

| Edukt | Ausbeute Diamin in % |
|---|---|
| Cyclohexylamin | 71 |
| n-Butylamin | 67 |
| Anilin | 65 |
| Hexylamin | 64 |
| Isopropylamin | 57 |
| Benzylamin | 55 |

Die hohen Diamin-Ausbeuten belegen, dass sich die erfindungsgemäße Umsetzung mit einer Vielzahl an unterschiedlichen, primären Aminen durchführen lässt.

### III. Unterschiedliches Verhältnis Dien:Amin

Das oben angegebene Beispiel wurde wie folgt abgeändert: 1 mmol Dicyclopentadien, n-Butylamin, 0,5 mol% [Rh(2-Ethylhexanoat)₃] (bezogen auf DCP) in 2-Ethylhexanol/2-Ethylhexansäure, 5 mL Toluol, 120 °C, 60 bar, CO/H₂, Reaktionszeit 2,5 h. Des Weiteren wurden zwei unterschiedliche Dien:Amin-Verhältnisse eingestellt. Der Umsatz der Diene lag sämtlich bei 100%. Es ergaben sich folgende Ausbeuten an di-sekundären Aminen:

| Verhältnis Dien : Amin | Ausbeute Diamin in % |
|---|---|
| 1 zu 6 | 66 |
| 1 zu 10 | 78 |

Das Konzentrationsverhältnis Dien zu Amin scheint großen Einfluss auf die Ausbeute an Diamin zu haben. Bevorzugterweise sollte zum Erzielen sehr hoher Ausbeuten mit einem mindestens 6-fachen oder besser noch mindestens 10-fachen Überschuss an primären Amin zu Dien gearbeitet werden. Des Weiteren zeigte sich überraschenderweise, dass unter hohen Aminkonzentrationen weniger Nebenreaktionen auftreten. Insbesondere können anscheinend durch diesen Parameter das Ausmaß unerwünschter Aldoladditionen gesteuert, d.h. reduziert werden.

### IV. Unterschiedliche Reaktionstemperaturen

Das oben angegebene Beispiel wurde wie folgt abgeändert: 1 mmol DCP, 6 mmol n-Butylamin, 0,5 mol% [Rh(octanoat)₂]₂ (bezogen auf DCP), 5 mL Toluol, 60 bar CO/H₂ (1:1), 350 Upm. Des Weiteren wurden drei unterschiedliche Reaktionstemperaturen eingestellt, wobei die Reaktionszeit bei 80°C 20 h und bei den beiden anderen Temperaturen 3 h betrug. Der Umsatz der Diene liegt bei den Versuchen über 100°C bei 100%. Es ergaben sich folgende Ausbeuten an di-sekundären Aminen:

| Reakti onstemperatur | Ausbeute Diamin in % |
|---|---|
| 80 | 0 |
| 110 | 25 |
| 120 | 88 |

Es zeigt sich, dass die Umsetzung zum Diamin unterhalb von 100°C sehr langsam abläuft, sodass selbst unter deutlicher Verlängerung der Reaktionszeit keine nennenswerten Umsätze zum Diamin detektiert werden können.

### V. Einsatz unterschiedlicher Lösungsmittel

Das oben angegebene Beispiel wurde in unterschiedlichen Lösungsmitteln durchgeführt. Der Umsatz der Diene liegt außer für Propylencarbonat (50%) und DMF (20%) sämtlich bei 100%. Es ergaben sich folgende Ausbeuten an di-sekundären Aminen:

| Lösungsmittel | Ausbeute Diamin in % |
|---|---|
| Toluol | 88 |
| Cyclohexan | 80 |
| Isopropanol | 60 |
| MeOH | 0 |
| Acetonitril | 0 |
| Propylencarbonat | 3 |
| DMF | 0 |

Anhand der gefundenen Ausbeuten wird ersichtlich, dass die Reaktion am besten in unpolaren Lösungsmitteln wie Toluol und Cyclohexan verläuft. Im Falle eines Einsatzes polarer Lösungsmittel finden sich unter den hier gewählten Reaktionsbedingungen geringere bis keine nennenswerten Umsätze zum Diamin. Die Ergebnisse der quantitativen Analyse zeigen, dass in diesen Fällen unter den gewählten Reaktionsbedingungen im Wesentlichen nur die erste Doppelbindung umgesetzt wird.

### VI. Einsatz unterschiedlicher Rh-Precursor-Konzentrationen

Das oben angegebene Beispiel wurde wie folgt abgeändert: 1 mmol Dicyclopentadien, 6 mmol n-Butylamin, Katalysator [Rh(2-Ethylhexanoat)₃] in 2-Ethylhexanol/2-Ethylhexansäure, 5 mL Toluol, 120°C, 60 bar, CO/H₂ 1:1, 2,5 h. Der Umsatz der Diene liegt sämtlich bei 100%. Es ergaben sich folgende Ausbeuten an di-sekundären Aminen:

| Katalysatorkonzentration in mol% (bezogen auf DCP) | Ausbeute Diamin in % |
|---|---|
| 0,25 | 17 |
| 0,50 | 48 |

Unter Verwendung der oben angegebenen Katalysatorkonzentrationen lassen sich innerhalb der hier gewählten Reaktionszeit und -Temperatur signifikante Umsätze zum Diamin erreichen. Insbesondere die höheren Rhodium-Precursorkonzentrationen sind bevorzugt, da diese zu insgesamt höheren Ausbeuten an gewünschtem Diamin führen. Des Weiteren zeigt sich, dass insbesondere die Kombination hoher Amin:Dien-Verhältnisse (beispielsweise größer als 6) und hoher Precursor-Konzentrationen (beispielsweise größer als 0,5 mol%) zu sehr hohen Ausbeuten an Diamin führen können. In dieser Kombination wird überraschenderweise auch die Bildung unerwünschter Nebenprodukte stark zurückgedrängt.

### VII. Einfluss unterschiedlicher Reaktionszeiten

Das oben angegebene Beispiel wurde wie folgt abgeändert: 16,6 mmol DCP, 99,6 mmol n-Butylamin, 0,5 mol% [Rh(octanoat)₂]₂ (bezogen auf DCP), 415 mmol Toluol, T = 120 °C, CO/H₂ 1:1, p = 60 bar. Der Umsatz der Diene liegt sämtlich bei 100%. Es ergaben sich folgende Ausbeuten an di-sekundären Aminen. Die Reaktionszeiten wurden wie folgt geändert:

| Reaktionszeit in min, T = 120 °C | Ausbeute Diamin in % |
|---|---|
| 20 | 7 |
| 40 | 31 |
| 60 | 66 |
| 80 | 92 |
| 240 | 92 |

Die erreichten Umsätze belegen, dass unter den gewählten Reaktionsbedingungen sich schon nach relativ kurzen Reaktionszeiten signifikante Umsätze an Diamin einstellen.

### VII. Einfluss unterschiedlicher Synthesegasdrücke

Das oben angegebene Beispiel wurde wie folgt abgeändert: 1 mmol DCP, 6 mmol n-Butylamin, 0,5 mol% [Rh(octanoat)₂]₂ (bezogen auf DCP), 5 mL Toluol, 120°C, CO/H₂ (1:1), 3 h; 350 Upm. Der Umsatz der Diene liegt sämtlich bei 100%. Es ergaben sich folgende Ausbeuten an di-sekundären Aminen

| Synthesegasdruck in bar | Ausbeute Diamin in % |
|---|---|
| 20 | 3 |
| 30 | 49 |
| 60 | 88 |

Im Rahmen der angegebenen Reaktionsbedingungen lassen sich insbesondere unter hohen Synthesegasdrücken ab 3 MPa, insbesondere ab 6 MPa, sehr hohe Ausbeuten an Diamin erhalten.

## Patentansprüche

1. Verfahren zur Synthese di-sekundärer Amine aus zyklischen Dienen und primären Aminen, **dadurch gekennzeichnet, dass** in nur einer Reaktionslösung zyklische Diene mit Synthesegas und primären Aminen an einem organophosphor-freien Metallkatalysator, der ausschließlich Rhodium als Katalysatormetall enthält, zur Reaktion gebracht werden, wobei die Reaktionslösung von weiteren stickstoffhaltigen organischen Verbindungen neben den als Edukt eingesetzten primären Aminen und den daraus entstehenden Produkten frei ist.

2. Verfahren nach Anspruch 1, wobei die Temperatur innerhalb der Umsetzung größer oder gleich 80°C und kleiner oder gleich 160°C beträgt.

3. Verfahren nach einem der vorhergehenden Ansprüche, wobei das Molverhältnis von eingesetztem primären Amin zu eingesetztem Dien (Amin:Dien) größer oder gleich 2 und kleiner oder gleich 40 beträgt.

4. Verfahren nach einem der vorhergehenden Ansprüche, wobei die Reaktion in Gegenwart eines Lösungsmittels durchgeführt wird.

5. Verfahren nach Anspruch 4, wobei als Lösungsmittel ein aprotisch unpolares Lösungsmittel eingesetzt wird.

6. Verfahren nach einem der vorhergehenden Ansprüche, wobei der Druck innerhalb der Umsetzung größer oder gleich 3 MPa und kleiner oder gleich 30 MPa beträgt.

7. Verfahren nach einem der vorhergehenden Ansprüche, wobei als Diene C5-C20 Diene mit mindestens einer zyklischen Struktur eingesetzt werden.

8. Verfahren nach einem der vorhergehenden Ansprüche, wobei als Diene bi-zyklische C7-C20 Diene eingesetzt werden.

9. Verfahren nach einem der Ansprüche 1 bis 7, wobei die Diene ausgesucht sind aus der Gruppe bestehend aus 1,4-Cyclohexadien, 4-Vinyl-1-Cyclohexen, 5-Vinyl-2-Norbonen, Norbornadien, Divinylbenzol, Dicyclopentadien, Tricyclopentadien oder Mischungen daraus.

10. Verfahren nach einem der vorhergehenden Ansprüche, wobei als Dien Dicyclopentadien eingesetzt wird.

11. Verfahren nach einem der vorhergehenden Ansprüche, wobei die primären Amine aus der Gruppe der primären aliphatischen oder araliphatischen C1-C18 Amine ausgesucht sind.

12. Verfahren nach einem der Ansprüche 1-10, wobei die primären Amine ausgesucht aus sind aus der Gruppe bestehend aus n-Butylamin, Isobutylamin, Cyclohexylamin, Benzylamin, Isopropylamin, Anilin, sowie Mischungen daraus.

13. Verfahren nach einem der vorhergehenden Ansprüche, wobei das molare Verhältnis CO zu H₂ (CO:H₂) des eingesetzten Synthesegases größer oder gleich 0,25 und kleiner oder gleich 2 beträgt.

14. Verfahren nach einem der vorhergehenden Ansprüche, wobei die Vorstufe des Rhodiumkatalysators ausgesucht ist aus der Gruppe bestehend aus [Rh(octanoat)₂]₂, [Rh(acac)(CO)₂], [Rh(2-Ethylhexanoat)₃], [Rh(2-Ethylhexanoat)₂]₂, [Rh(acac)(cod)], [Rh(cod)Cl]₂, [Rh(acac)₃], [Rh(cod)₂]BF₄ oder Mischungen daraus.

15. Verfahren nach einem der vorhergehenden Ansprüche, wobei die molare Menge des Rhodiumkatalysators bezogen auf das Dien (Katalysator:Dien) größer oder gleich 0,0001 mol-% und kleiner oder gleich 1,0 mol-% beträgt.

## Claims

1. Method for the synthesis of di-secondary amines from cyclic dienes and primary amines, **characterized in that** in only one reaction solution cyclic dienes are reacted with synthesis gas and primary amines at an organophosphorus-free metal catalyst which contains only rhodium as catalyst metal, wherein the reaction solution besides the primary amines used as a starting material and the products resulting therefrom is free of further nitrogen-containing organic compounds.

2. Method according to claim 1, wherein the temperature during the reaction is greater than or equal to 80°C and less than or equal to 160°C.

3. Method according to any one of the preceding claims, wherein the molar ratio of primary amine used to diene used (amine:diene) is greater than or equal to 2 and less than or equal to 40.

4. Method according to any one of the preceding claims, wherein the reaction is carried out in the presence of a solvent.

5. Method according to claim 4, wherein an aprotic non-polar solvent is used as the solvent.

6. Method according to any one of the preceding claims, wherein the pressure during the reaction is greater than or equal to 3 MPa and less than or equal to 30 MPa.

7. Method according to any one of the preceding claims, wherein as dienes C5 to C20 dienes with at least one cyclic structure are used.

8. Method according to any one of the preceding claims, wherein bicyclic C7 to C20 dienes are used as dienes.

9. Method according to any one of claims 1 to 7, wherein the dienes are selected from the group consisting of 1,4-cyclohexadiene, 4-vinyl-1-cyclohexene, 5-vinyl-2-norbonene, norbornadiene, divinylbenzene, dicyclopentadiene, tricyclopentadiene or mixtures thereof.

10. Method according to any one of the preceding claims, wherein dicyclopentadiene is used as diene.

11. Method according to any one of the preceding claims, wherein the primary amines are selected from the group of primary aliphatic or araliphatic C1 to C18 amines.

12. Method according to any one of claims 1 to 10, wherein the primary amines are selected from the group consisting of n-butylamine, isobutylamine, cyclohexylamine, benzylamine, isopropylamine, aniline, and mixtures thereof.

13. Method according to any one of the preceding claims, wherein the molar ratio of CO to H₂ (CO:H₂) of the synthesis gas used is greater than or equal to 0.25 and less than or equal to 2.

14. Method according to any one of the preceding claims, wherein the precursor of the rhodium catalyst is selected from the group consisting of [Rh(octanoate)₂]₂, [Rh(acac)(CO)₂], [Rh(2-Ethylhexanoate)₃], [Rh(2-Ethylhexanoate)₂]₂, [Rh(acac)(cod)], [Rh(cod)Cl]₂, [Rh(acac)₃], [Rh(cod)₂]BF₄ or mixtures thereof.

15. Method according to any one of the preceding claims, wherein the molar amount of the rhodium catalyst with respect to the diene (catalyst:diene) is greater than or equal to 0.0001 mol-% and less than or equal to 1.0 mol-%.

## Revendications

1. Procédé de synthèse d'amines disecondaires à partir de diènes cycliques et d'amines primaires, **caractérisé en ce que** des diènes cycliques sont mis à réagir uniquement dans une solution réactionnelle avec du gaz de synthèse et des amines primaires sur un catalyseur métallique exempt de composé phosphoré organique, qui contient exclusivement du rhodium en tant que métal de catalyseur, où la solution réactionnelle est exempte d'autres compositions organiques contenant de l'hydrogène à côté des amines primaires engagées en tant que produits de départ et des produits en résultant.

2. Procédé selon la revendication 1, dans lequel la température dans la conversion est égale ou supérieure à 80 °C et inférieure ou égale à 160 °C.

3. Procédé selon l'une des revendications précédentes, dans lequel le rapport molaire de l'amine primaire engagée sur le diène engagé (amine : diène) est égal ou supérieur à 2 et inférieur ou égal à 40.

4. Procédé selon l'une des revendications précédentes, dans lequel la réaction est effectuée en présence d'un solvant.

5. Procédé selon la revendication 4, dans lequel un solvant non polaire aprotique est engagé en tant que solvant.

6. Procédé selon l'une des revendications précédentes, dans lequel la pression dans la conversion est égale ou supérieure à 3 MPa et inférieure ou égale à 30 MPa.

7. Procédé selon l'une des revendications précédentes, dans lequel des diènes en C5 à C20 avec au moins une structure cyclique sont engagés en tant que diènes.

8. Procédé selon l'une des revendications précédentes, dans lequel des diènes en C7 à C20 bicycliques sont engagés en tant que diènes.

9. Procédé selon l'une des revendications 1 à 7, dans lequel les diènes sont sélectionnés dans le groupe constitué du 1,4-cyclohexadiène, du 4-vinyl-1-cyclohexène, du 5-vinyl-2-norbornène, du norbornadiène, du divinylbenzène, du dicyclopentadiène, du tricyclopentadiène ou de mélanges de ceux-ci.

10. Procédé selon l'une des revendications précédentes, dans lequel du dicyclopentadiène est engagé en tant que diène.

11. Procédé selon l'une des revendications précédentes, dans lequel les amines primaires sont sélectionnées dans le groupe des amines en C1 à C18 aliphatiques primaires ou araliphatiques.

12. Procédé selon l'une des revendications 1 à 10, dans lequel les amines primaires sont sélectionnées dans le groupe constitué de la n-butylamine, de l'isobutylamine, de la cyclohexylamine, de la benzylamine, de l'isopropylamine, de l'aniline, ainsi que de mélanges de celles-ci.

13. Procédé selon l'une des revendications précédentes, dans lequel le rapport molaire CO sur H₂ (CO : H₂) du gaz de synthèse engagé est égal ou supérieur à 0,25 et inférieur ou égal à 2.

14. Procédé selon l'une des revendications précédentes, dans lequel le précurseur du catalyseur de rhodium est choisi dans le groupe constitué du rhodium (II) octanoate dimère [Rh(octaneoate)₂]₂, de l'acétyl acénato dicarbonyl rhodium [Rh(acac) (CO)₂], du rhodium (III) 2-éthyl hexanoate [Rh(2-éthylhexanoate)₃], du rhodium (II) 2-éthyl hexanoate dimère [Rh(2-éthyl hexanoate)₂]₂, de l'acétyl acénato cyclo octadiène rhodium [Rh(acac) (cod)], du chlorure de cyclo octadiène rhodium dimère [Rh(codC1]₂, du rhodium acétyl acénatonate [Rh(acac)₃], du bis cyclo octadiène rhodium tétrafluoroborate [Rh(cod)₂]BF₄ ou de mélanges de ceux-ci.

15. Procédé selon l'une des revendications précédentes, dans lequel la quantité molaire du catalyseur au rhodium par rapport au diène (catalyseur : diène) est égale ou supérieure à 0,0001 % en mole et inférieure ou égale à 1,0 % en mole.
